# EUROPEAN PATENT SPECIFICATION

(11) **EP 1 621 609 B1**
(45) Date of publication and mention of the grant of the patent: **23.12.2009**
(21) Application number: 05016615.6
(22) Date of filing: 29.07.2005
(51) Int. Cl.: C12N 1/20, C02F 3/34

(54) **PVA-decomposing bacteria and method for decomposing PVA**
PVA-zersetzendes Bakterium und Methode für das Zersetzen von PVA
Bactérie décomposant l'alcool de polyvinylique (PVA) et procédé pour décomposer PVA

(30) Priority: 30.07.2004 JP 2004223954
(43) Date of publication of application: 01.02.2006
(73) Proprietor: FUJIFILM Corporation, Minato-ku Tokyo (JP)
(72) Inventor: Kojima, Masayoshi, Minami-ashigara-shi Kanagawa 250-0193 (JP)
(74) Representative: HOFFMANN EITLE

(56) References cited:
- DATABASE WPI Section Ch, Week 199525 Derwent Publications Ltd., London, GB; Class A14, AN 1995-190360 XP002351864 & JP 07 108297 A (AGENCY OF IND SCI & TECHNOLOGY) 25 April 1995 (1995-04-25)
- TOKIWA YUTAKA ET AL: "A modified method for isolating poly(vinyl alcohol)-degrading bacteria and study of their degradation patterns" BIOTECHNOLOGY LETTERS, KEW, SURREY, GB, vol. 23, no. 23, December 2001 (2001-12), pages 1937-1941, XP002331739 ISSN: 0141-5492
- MORI TATSUMA ET AL: "Isolation and characterization of a strain of Bacillus megaterium that degrades poly(vinyl alcohol)" BIOSCIENCE BIOTECHNOLOGY AND BIOCHEMISTRY, vol. 60, no. 2, 1996, pages 330-332, XP008054707 ISSN: 0916-8451
- KIM B C ET AL: "Degradation of polyvinyl alcohol by Sphingomonas sp. SA3 and its symbiote" JOURNAL OF INDUSTRIAL MICROBIOLOGY AND BIOTECHNOLOGY, BASINGSTOKE, GB, vol. 30, no. 1, 1 January 2003 (2003-01-01), pages 70-74, XP002331737 ISSN: 1367-5435

## Description

### Technical Field

The present invention relates to decomposition and assimilation of polyvinyl alcohol (hereafter abbreviated as "PVA").

### Background Art

PVA is widely used for industrial applications as a water-soluble synthetic polymer. It is extensively employed as, for example, a starting material of a synthetic fiber or film, a fiber-processing agent, a paper-processing agent, an adhesive, a binder for inorganic substances, or a polymerization stabilizer for vinyl chloride resins.

Recently, concerns over environmental issues have been enhanced on a global scale, and biodegradable polymers that are decomposed by microorganisms or enzymes in nature have been actively studied. Also, a technique of decomposing various types of compounds before they are released in the natural environment to thereby decrease adverse impacts on environment has drawn attention. PVA waste liquid has been heretofore considered to be a cause of elevated COD levels in wastewater, treatment of PVA waste liquid with microorganisms such as biological sludge has been examined, and decomposing bacteria have been searched for. Examples of microorganisms that are known to be capable of decomposing PVA include *Pseudomonas* bacteria, *Enterobacter* bacteria, *Acinetobacter* bacteria, *Corynebacterium* bacteria, *Rhodococcus* bacteria, *Caseobacter* bacteria, and *Sphingomonas* bacteria (for example, JP Patent Publication (Kokoku) Nos. 55-1791 B (1980), 57-27713 B (1982), and 57-27714 B (1982) and JP Patent Publication (Kokai) Nos. 8-140667 A (1996), 7-108297 A (1995), and 2003-250527 A).

The decomposition of PVA by using Gram-positive bacteria has been disclosed for microorgansims belonging to the genus *Cornynebacterium, Caseobacter* and/or *Rhodococcus.* A method for treating waste water containing PVA with these bacteria has also been described (JP 7108297).

Tokiwa Yutaka et al. describe a modified method for isolating poly(vinyl alcohol)-degrading bacteria and study of their degradation patterns (Biotechnology Letters, vol. 23, no. 23, December 2001, pp. 1937-1941).

Mori Tatsuma et al. describe the isolation and characterisation of a strain of *Bacillus megaterium* that degrades poly(vinyl alcohol) (Bioscience Biotechnology, vol. 60, no. 2, 1996, pp. 330-332).

### Disclosure of the Invention

The object of the present invention is to provide PVA-decomposing bacteria that are independently highly capable of decomposing and assimilating various types of PVA derivatives, and a method for decomposing and assimilating PVA with the utilization of such bacteria, and to provide a method for decomposing and assimilating PVA wherein microorganisms containing such bacteria exhibit a high level of PVA decomposing activity even in the presence of other nutrients, such as gelatin and starch, that are easily assimilable by microorganisms.

The present inventors have extensively searched for microorganisms in nature, such as in rivers, soil, and biological sludge. As a result, they have found that *Microbacterium laevaniformans* separated from biological sludge sampled from the Fuji Photo Film Fujinomiya Factory are highly capable of decomposing and assimilating PVA and derivatives thereof, and use thereof enables efficient cleanup treatment of various types of PVA-containing wastes and the like. They have also found that even when nutrients such as gelatin and starch, which are easily assimilable by microorganisms, are present in wastes, the wastes can be effectively cleaned up while decomposition of PVA remains undelayed.

Thus, the present invention provides a method for decomposing polyvinyl alcohol or a polyvinyl alcohol derivative according to claim 1.

According to another aspect, the present invention provides a method for cleaning up wastewater, waste liquid or soil, according to claim 2.

According to further another aspect, the present invention provides polyvinyl alcohol-decomposing bacteria which are deposited as *Microbacterium laevaniformans* KSS-11 (accession number: FERM BP-10355).

According to further another aspect, the present invention provides a method for decomposing polyvinyl alcohol or a polyvinyl alcohol derivative, which comprises immobilizing microorganisms containing *Microbacterium laevaniformans* KSS-11 (accession number: FERM BP-10355) on a carrier and bringing it into contact with polyvinyl alcohol or a polyvinyl alcohol derivative.

According to further another aspect, the present invention provides a method for cleaning up wastewater, waste liquid or soil, which comprises immobilizing microorganisms containing *Microbacterium laevaniformans* KSS-11 (accession number: FERM BP-10355) on a carrier and bringing it into contact with wastewater, waste liquid or soil containing polyvinyl alcohol or a polyvinyl alcohol derivative.

Preferably, the bacteria-immobilized carrier is comprised of active carbons or crosslinked polymers.

### Brief Description of the Drawings

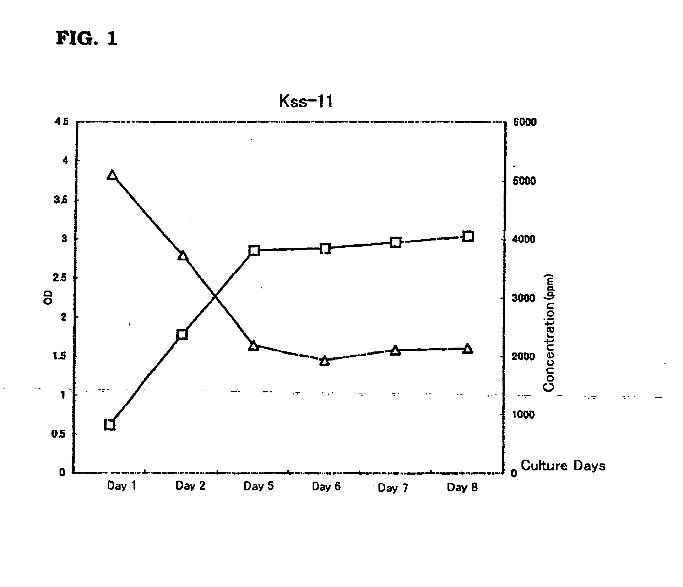

Fig. 1 shows the results of assaying PVA decomposition and assimilation with the utilization of *Microbacterium laevaniformans* KSS-11 strain. The squares indicate the concentration of the strain, and the triangles indicate the concentration of PVA.

### Best Modes for Carrying out the Invention

Examples of PVA that is used in the present invention include PVA obtained by saponification of polyvinyl acetate, as well as functional PVA derivatives such as anionic, cationic, silane-reactive, or terminal-hydrophobic PVA. PVA can be decomposed and assimilated by using the bacteria of the present invention regardless of the degree of PVA polymerization or the degree of saponification, i.e., whether complete or partial saponification is involved. Examples of highly degradable PVA include those having a degree of polymerization of 300 to 3,500 and a degree of saponification of 86% to 99%. Examples of PVA derivatives include anionic PVA and terminal hydrophobic PVA.

The bacteria that are capable of decomposing and assimilating polyvinyl alcohol according to the present invention belong to the genus *Microbacterium,* and are preferably strains which belong to the species *Microbacterium laevaniformans*.

The genus *Microbacterium* which is capable of decomposing and assimilating polyvinyl alcohol, has been extensively searched for in nature. For example, it can be separated in the following manner.

The genus *Microbacterium* which is capable of decomposing and assimilating polyvinyl alcohol according to the present invention, for example, the species *Microbacterium laevaniformans,* is separated, for example, in the following manner. Specifically, samples such as those from rivers, biological sludge, soil, marine water or lake water are filtered through a membrane filter (pore diameter: 0.1 µm to 0.5 µm), and the membrane filter is soaked in a PVA liquid medium and subjected to shaking culture to evaluate the presence of PVA-decomposing bacteria. Thereafter, the culture solution is dispersed on an adequate medium to form colonies. The resulting colonies are inoculated on a low nutrient medium containing PVA, and bacteria capable of decomposing PVA may be selected and then separated.

An example of the bacteria capable of decomposing and assimilating PVA and derivatives thereof according to the present invention is *Microbacterium laevaniformans* KSS-11 strain. This *Microbacterium laevaniformans* KSS-11 strain is deposited at the International Patent Organism Depositary of the National Institute of Advanced Industrial Science and Technology (Tsukuba Central 6, 1-1-1 Higashi, Tsukuba, Ibaraki, Japan) as of June 16, 2004, under the accession number FERM P-20081, and was transferred to International deposition under Budapest treaty as of June 23, 2005 under the accession number FERM BP-10355. Mycological properties and the results of genetic analysis of the *Microbacterium laevaniformans* KSS-11 are described below.

### (Mycological properties)

Properties of medium: LB agar medium (Sambrook, J., E. F. Fritsch, and T. Maniatis, 1989, Molecular cloning: a laboratory manual, 2nd ed., Cold Spring Harbor Laboratory Press, N.Y.); yellow, slightly convex, and round colonies are formed.

### (Genetic analysis)

### Analysis of 16 SrDNA

The KSS-11 strain was inoculated on LB agar medium, DNA was extracted from a culture product at 30°C, the extracted DNA was amplified by PCR, and the PCR product was purified. Thereafter, cycle sequencing was carried out using the BigDye™ Terminator v. 3.1 (Applied Biosystems) to determine the nucleotide sequence of 16 SrDNA, and 16 SrDNA was analyzed using the FASTA Program of the DNA Data Bank of Japan (http://www.ddbj.nig.ac.jp/) on the web. As a result, the 16 SrDNA of the KSS-11 strain of the present invention was found to have 98.0% homology to that of *Microbacterium laevaniformans* (DDBJ/EMBL/GenBank databases direct submittion. Accession No.AB007997).

In principle, the aforementioned bacteria are cultured in accordance with, for example, a culture technique for microorganisms that belong to the genus *Microbacterium.* In general, bacteria are preferably cultured under aerobic conditions, such as with shaking culture via liquid culture or aeration agitation culture.

A variety of synthetic media, semi-synthetic media, naturally-occurring media, and the like for aerobic bacteria can be employed for culture. Examples thereof include meat extract and peptone.

The pH level of a medium is preferably between 6 and 8. The culture temperature is preferably kept at a temperature where the genus *Microbacterium* is grown well. This is generally about 15°C to 42°C, and particularly preferably about 25°C to 38°C. A culture period is generally for 2 days to 1 week under aerobic conditions regardless of the type of culture conducted, i.e., whether liquid shaking culture or aeration agitation culture is employed. It should be noted that such culture conditions can be adequately altered depending on the types and properties of microorganisms to be used, external conditions, and other conditions.

The bacteria according to the present invention can effectively decompose and assimilate PVA and PVA derivatives as described in the Examples below. Accordingly, bacteria that belong to the genus *Microbacterium* (for example, *Microbacterium laevaniformans* ), microorganisms containing the same, or bacterial components thereof can be brought into contact with industrial wastes, such as household effluents, industrial effluents, or waste liquids, or PVA-containing substances such as soil, to clean up them. The bacterial components include cultured products of bacteria that belong to the genus *Microbacterium,* immobilized bacteria, bacterial extracts, immobilized bacterial extracts, bacterial liquid extracts, culture solutions, and culture filtrates. Examples of carriers that can be used for immobilizing bacteria include active carbons and crosslinked polymers.

Examples of methods for decomposing PVA with the utilization of the microorganisms according to the present invention in the case of wastewater cleanup include a means for installing a processor having the genus *Microbacterium* of the present invention or microorganisms containing the same immobilized thereon, at individual sources, and a means for adding the bacteria of the present invention or microorganisms containing the same to an activated sludge tank or the like in a wastewater plant. An example of such method in the case of soil cleanup is a means for applying the bacteria of the present invention or microorganisms containing the same to soil. In the case of environmental cleanup, a means for applying the bacteria of the present invention or microorganisms containing the same to river or lake in nature can be employed. Such PVA decomposition treatment is carried out at a pH 4 to 10 and preferably pH 5 to 8, and at a processing temperature of 5°C to 40°C and preferably of 15°C to 35°C, for 0.5 hour to 14 days in general and preferably for 1 hour to 7 days.

The present invention is hereafter described in greater detail with reference to the following examples, although the technical scope of the present invention is not limited thereto.

### Examples

### Example 1: Isolation of Microbacterium laevaniformans KSS-11 strain

100 ml of PVA medium (Table 1) was prepared, wild-type *E*. *coli* MG1655 strain having no PVA decomposition ability was inoculated in order to remove impurities contained in PVA used as a starting material, such as acetate, and shaking culture was carried out at 28°C for 1 day. The culture solution was filtered through a membrane filter (pore diameter: 0.2 µm) for sterilization. A suspension of PVA waste sludge (50 µl) was inoculated on the filtered PVA culture medium, and shaking culture was carried out at 28°C for 8 days. LB agar medium was coated with a part of the culture solution, the resultant was cultured at 28°C for 5 days, single colonies were selected from a plate on which colony growth had been observed, and the ability for PVA decomposition was confirmed by using the filtered PVA culture medium, and the identification test were carried out. Thus, *Microbacterium laevaniformans* KSS-11 strain was obtained.

**Table 1: PVA 0.5% medium**

| Components | Weight(g/l) |
|---|---|
| PVA500 | 5 |
| (NH₄) ₂SO₄ | 1 |
| KH₂PO₄ | 0.4 |
| K₂HPO₄ | 3.2 |
| MgSO₄ | 0.98 |
| NaCl | 0.1 |
| FeSO₄ · 7H₂O | 0.01 |
| Yeast extract | 1 |

### Example 2: Decomposition and assimilation of PVA utilizing Microbacterium laevaniformans KSS-11 strain

KSS-11 strains grown in an LB agar medium were inoculated on 10 ml of 0.5% PVA medium using an inoculating loop, and shaking culture was carried out at 28°C for 8 days. Part of the culture solution was removed with the elapse of time, and turbidity (OD 600) and PVA concentration thereof were measured. PVA concentration was measured in accordance with the method described in Kim, B. C., C. K. Sohn, S. K. Lim, J. W. Lee, and W. Park, 2003, Degradation of polyvinyl alcohol by Sphingomonas sp. SA3 and its symbiote, J. Ind. Microbiol. Biotechnol., 30, 70-74. As a result, it was shown that the KSS-11 strain was capable of effectively decomposing PVA by itself (Fig.1).

### Effect of the Invention

The method for decomposing PVA according to the present invention is effective for decomposition and assimilation of PVA in the clean up and composting treatment of various forms of wastewater, waste liquid soil, and the like.

## Claims

1. A method for decomposing polyvinyl alcohol or a polyvinyl alcohol derivative, which comprises bringing *Microbacterium laevaniformans* KSS-11 with the accession number: FERM BP-10355, or components of said microbacterium capable of decomposing polyvinyl alcohol or a polyvinylalcohol derivative into contact with polyvinyl alcohol or a polyvinyl alcohol derivative.

2. A method for cleaning up wastewater, waste liquid or soil, which comprises bringing *Microbacterium laevaniformans* KSS-11 with the accession number: FERM BP-10355, or components of said microbacterium capable of decomposing polyvinyl alcohol or a polyvinylalcohol derivative into contact with wastewater, waste liquid or soil containing polyvinyl alcohol or a polyvinyl alcohol derivative.

3. Polyvinyl alcohol-decomposing bacteria deposited as *Microbacterium laevaniformans* KSS-11 with the accession number: FERM BP-10355.

4. A method for decomposing polyvinyl alcohol or a polyvinyl alcohol derivative, which comprises immobilizing *Microbacterium laevaniformans* KSS-11 with the accession number FERM BP-10355 on a carrier and bringing it into contact with polyvinyl alcohol or a polyvinyl alcohol derivative.

5. A method for cleaning up wastewater, waste liquid or soil, which comprises immobilizing *Microbacterium laevaniformans* KSS-11 with the accession number FERM BP-10355 on a carrier and bringing it into contact with wastewater, waste liquid or soil containing polyvinyl alcohol or a polyvinyl alcohol derivative.

6. The method according to claim 4 or 5, wherein the bacteria-immobilized carrier is comprised of active carbons or crosslinked polymers.

## Patentansprüche

1. Verfahren zum Abbau von Polyvinylalkohol oder einem Polyvinylalkoholderivat, das umfasst, Microbacterium laevaniformans KSS-11 mit der Hinterlegungsnummer: FERM BP-10355 oder Bestandteile dieses Mikrobakteriums, das die Fähigkeit besitzt, Polyvinylalkohol oder ein Polyvinylalkoholderivat abzubauen, mit Polyvinylalkohol oder einem Polyvinylalkoholderivat in Kontakt zu bringen.

2. Verfahren zur Reinigung von Abwasser, flüssigem Abfall oder Boden, das umfasst, Microbacterium laevaniformans KSS-11 mit der Hinterlegungsnummer: FERM BP-10355 oder Bestandteile dieses Mikrobakteriums, das die Fähigkeit besitzt, Polyvinylalkohol oder ein Polyvinylalkoholderivat abzubauen, mit Abwasser, flüssigem Abfall oder Boden, das/der Polyvinylalkohol oder ein Polyvinylalkoholderivat enthält, in Kontakt zu bringen.

3. Polyvinylalkohol-abbauende Bakterien, hinterlegt als Microbacterium laevaniformans KSS-11 mit der Hinterlegungsnummer: FERM BP-10355.

4. Verfahren zum Abbau von Polyvinylalkohol oder einem Polyvinylalkoholderivat, das umfasst, Microbacterium laevaniformans KSS-11 mit der Hinterlegungsnummer: FERM BP-10355 auf einem Träger zu immobilisieren und mit Polyvinylalkohol oder einem Polyvinylalkoholderivat in Kontakt zu bringen.

5. Verfahren zur Reinigung von Abwasser, flüssigem Abfall oder Boden, das umfasst, Microbacterium laevaniformans KSS-11 mit der Hinterlegungsnummer: FERM BP-10355 auf einem Träger zum immobilisieren und mit Abwasser, flüssigem Abfall oder Boden, das/der Polyvinylalkohol oder ein Polyvinylalkoholderivat enthält, in Kontakt zu bringen.

6. Verfahren gemäß Anspruch 4 oder 5, wobei der Bakterienimmobilisierte Träger aktive Kohlenstoffe oder quervernetzte Polymere umfasst.

## Revendications

1. Une méthode pour décomposer de l'alcool polyvinylique ou un dérivé de l'alcool polyvinylique, qui comprend la mise en contact de *Microbacterium laevaniformans* KSS-11 ayant le numéro d'accès FERM BP-10355, ou des composés dudit microbacterium capables de décomposer de l'alcool polyvinylique ou un dérivé de l'alcool polyvinylique, avec de l'alcool polyvinylique ou un dérivé de l'alcool polyvinylique.

2. Une méthode pour nettoyer des eaux usées, un liquide ou un sol usagés, qui comprend la mise en contact de *Microbacterium laevaniformans* KSS-11 ayant le numéro d'accès FERM BP-10355, ou des composés dudit microbacterium capables de décomposer de l'alcool polyvinylique ou un dérivé de l'alcool polyvinylique, avec des eaux usées, un liquide ou un sol usagés contenant de l'alcool polyvinylique ou un dérivé de l'alcool polyvinylique.

3. Une bactérie décomposant l'alcool polyvinylique déposée en tant que *Microbacterium laevaniformans* KSS-11 ayant le numéro d'accès FERM BP-10355.

4. Une méthode pour décomposer l'alcool polyvinylique ou un dérivé de l'alcool polyvinylique, qui comprend l'immobilisation de *Microbacterium laevaniformans* KSS-11 ayant le numéro d'accès FERM BP-10355 sur un support, et sa mise en contact avec de l'alcool polyvinylique ou un dérivé de l'alcool polyvinylique.

5. Une méthode pour nettoyer des eaux usées, un liquide ou un sol usagés, qui comprend l'immobilisation de *Microbacterium laevaniformans* KSS-11 ayant le numéro d'accès FERM BP-10355 sur un support, et sa mise en contact avec des eaux usées, un liquide ou un sol usagés contenant de l'alcool polyvinylique ou un dérivé de l'alcool polyvinylique.

6. La méthode selon la revendication 4 ou 5, dans laquelle le support sur lequel sont immobilisées des bactéries est constitué de charbon activé ou de polymères réticulés.
